# EUROPEAN PATENT APPLICATION

(11) **EP 3 603 542 A1**
(43) Date of publication of application: **05.02.2020**
(21) Application number: 19189865.9
(22) Date of filing: 02.08.2019
(51) Int. Cl.: A61B 17/128

(54) **ROTATION KNOB ASSEMBLIES AND SURGICAL INSTRUMENTS INCLUDING THE SAME**

(30) Priority: 03.08.2018 US 201862714203 P; 10.05.2019 US 201916408497
(71) Applicant: Covidien LP, Mansfield, MA 02048 (US)
(72) Inventor: BARIL, Jacob, C., Norwalk, Connecticut 06851 (US); MEEHAN, Christopher M., New Haven, Connecticut 06511 (US); CRESTON, Brian J., Madison, Connecticut 06443 (US); ADDI, Ernest A., Middletown, Connecticut 06457 (US); ZAMMATARO, Thomas A., Hamden, Connecticut 06517 (US); KUNG, Amy, Hamden, Connecticut 06517 (US); DININO, Matthew A., Newington, Connecticut 06111 (US); THOMAS, Justin, New Haven, Connecticut 06512 (US); PILLETERE, Roy J., North Haven, Connecticut 06473 (US)
(74) Representative: Soames, Candida Jane

(57) **Abstract**

A rotation knob assembly, handle assembly including the same, and surgical instrument including the same are disclosed. An outer knob of the assembly defining a longitudinal lumen and at least one transverse aperture having a threaded portion and a smooth portion. An inner sleeve of the assembly includes a body defining a longitudinal lumen and at least one transverse. At least one stepped pin of the assembly includes a body portion disposed within the smooth portion of the at least one outer knob transverse aperture and a tip portion disposed within the at least one inner sleeve transverse aperture to fix the outer knob and the inner sleeve with one another. At least one screw of the assembly is threadingly engaged within the threaded portion of the at least one outer knob transverse aperture to retain the outer knob and the inner sleeve fixed with one another.

## Description

### CROSS-REFERENCE TO RELATED APPLICATIONS

This application claims the benefit of, and priority to, U.S. Provisional Patent Appl. No. 62/714,203, filed August 3, 2018, the entire contents of which is hereby incorporated herein by reference.

### TECHNICAL FIELD

The present disclosure relates to surgical instruments such as, for example, surgical clip appliers. More particularly, the present disclosure relates to rotation knob assemblies for surgical clip appliers and surgical clip appliers including the same.

### BACKGROUND

Surgical clip appliers are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures.

Endoscopic surgical clip appliers having various sizes (e.g., diameters), that are configured to apply a variety of diverse surgical clips and are capable of applying a single or multiple surgical clips during an entry to the body cavity. Such surgical clips are typically fabricated from a biocompatible material and are usually compressed over tissue. Once applied to tissue, the compressed surgical clip terminates the flow of fluid therethrough.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

Provided in accordance with aspects of the present disclosure is a rotation knob assembly for a surgical instrument including an outer knob, an inner sleeve, at least one stepped pin, and at least one screw.

The outer knob of the rotation knob assembly defines an outer knob lumen extending longitudinally therethrough and at least one outer knob transverse aperture extending transversely therethrough into communication with the outer knob lumen. The at least one outer knob transverse aperture includes a threaded portion and a smooth portion that defines a first diameter.

The inner sleeve of the rotation knob assembly is disposed within the outer knob lumen and includes a body defining an inner sleeve lumen extending longitudinally therethrough and at least one inner sleeve transverse aperture extending transversely through the body into communication with the inner sleeve lumen. The at least one inner sleeve transverse aperture defines a second diameter less than the first diameter.

The at least one stepped pin of the rotation knob assembly includes a body portion disposed within the smooth portion of the at least one outer knob transverse aperture and a tip portion disposed within the at least one inner sleeve transverse aperture to fix the outer knob and the inner sleeve with one another.

The at least one screw of the rotation knob assembly is threadingly engaged within the threaded portion of the at least one outer knob transverse aperture to retain the at least one stepped pin in position, thereby retaining the outer knob and the inner sleeve fixed with one another.

In an aspect of the present disclosure, the rotation knob assembly further includes an intermediate collar disposed between the outer knob and the inner sleeve.

In another aspect of the present disclosure, the intermediate collar is fixed and the outer knob and the inner sleeve are together rotatable relative to the intermediate collar.

In still another aspect of the present disclosure, the outer knob lumen includes a proximal lumen portion and a distal lumen portion. The proximal lumen portion defines a diameter greater than a diameter of the distal lumen portion and receives the intermediate collar therein.

In another aspect of the present disclosure, the rotation knob assembly further includes at least one spring disposed within the at least one outer knob transverse aperture. The at least one spring is compressed between the at least one stepped pin and the at least one screw.

In yet another aspect of the present disclosure, the outer knob defines a plurality of grooves disposed on an interior surface thereof surrounding the outer knob lumen. At least one groove of the plurality of grooves is configured to receive a corresponding indexing protrusion of an elongated assembly inserted into the outer knob to rotationally fix the elongated assembly relative to the outer knob.

In still yet another aspect of the present disclosure, the at least one outer knob transverse aperture further defines a seat and the at least one screw includes a head configured to be received within the seat.

A handle assembly of a surgical instrument provided in accordance with aspects of the present disclosure includes a housing, a drive assembly, a trigger, and a rotation knob assembly. The housing defines a body portion, a fixed handle portion depending from the body portion, and a distal nose extending distally from the body portion. The drive assembly is disposed within the housing. The trigger is pivotably connected to the housing and operably associated with the drive assembly. The trigger is movable relative to the fixed handle portion of the housing from an un-actuated position to an actuated position to actuate the drive assembly. The rotation knob assembly extends distally from the distal nose of the housing and may be configured similarly to any of the aspects detailed hereinabove or otherwise herein.

In aspects where the rotation knob assembly includes an intermediate collar disposed between the outer knob and the inner sleeve, the intermediate collar may be fixed relative to the distal nose of the housing while the outer knob and the inner sleeve are together rotatable relative to the intermediate collar and the distal nose of the housing.

In an aspect of the present disclosure, the rotation knob assembly further includes at least one spring disposed within the at least one outer knob transverse aperture. The at least one spring is compressed between the at least one stepped pin and the at least one screw.

In another aspect of the present disclosure, the handle assembly further includes a latch assembly operably associated with the housing and configured to releasably engage an elongated assembly inserted through the rotation knob assembly and into the distal nose of the housing.

A surgical instrument provided in accordance with aspects of the present disclosure includes a handle assembly configured similarly to any of the aspects detailed hereinabove or otherwise herein (and including a rotation knob assembly configured similarly to any of the aspects detailed hereinabove or otherwise herein), and an elongated assembly extending distally from the handle assembly and supporting an end effector assembly at a distal end portion thereof.

### BRIEF DESCRIPTION OF THE DRAWINGS

Aspects and features of the presently-disclosed rotation knob assemblies for surgical clip appliers and surgical clip appliers including the same are described in detail with reference to the drawing figures wherein like reference numerals identify similar or identical structural elements and:
FIG. 1 is a front, perspective view of a surgical clip applier provided in accordance with the present disclosure including a handle assembly having an elongated assembly engaged therewith;
FIG. 2 is front, perspective view of the surgical clip applier of FIG. 1 with the elongated assembly removed from the handle assembly;
FIG. 3A is a side, perspective view of a distal end portion of the elongated assembly of FIGS. 1 and 2;
FIG. 3B is a side, perspective view of a distal end portion of another elongated assembly configured for use with the surgical clip applier of FIG. 1;
FIG. 4 is an enlarged, longitudinal, cross-sectional view of a portion of the handle assembly of the surgical clip applier of FIG. 1 including the elongated assembly of FIG. 1 engaged therewith;
FIG. 5 is an enlarged, transverse, cross-sectional view taken across section line "5-5" in Fig. 4;
FIG. 6 is a perspective view of a rotation knob assembly of the handle assembly of the surgical clip applier of FIG. 1;
FIG. 7 is an exploded, perspective view of the rotation knob assembly of FIG. 6;
FIG. 8 is a perspective, longitudinal, partial cross-sectional view of the rotation knob assembly of FIG. 6;
FIG. 9 is an exploded, perspective view of a rotation knob assembly provided in accordance with the present disclosure for use in the handle assembly of the surgical clip applier of FIG. 1; and
FIG. 10 is a perspective, longitudinal partial cross-sectional view of the rotation knob assembly of FIG. 9.

### DETAILED DESCRIPTION

The present disclosure provides rotation knob assemblies for surgical instruments and surgical instruments including the same. Although detailed herein as incorporated into a surgical clip applier, the rotation knob assemblies of the present disclosure may alternatively be incorporated into any suitable surgical instrument.

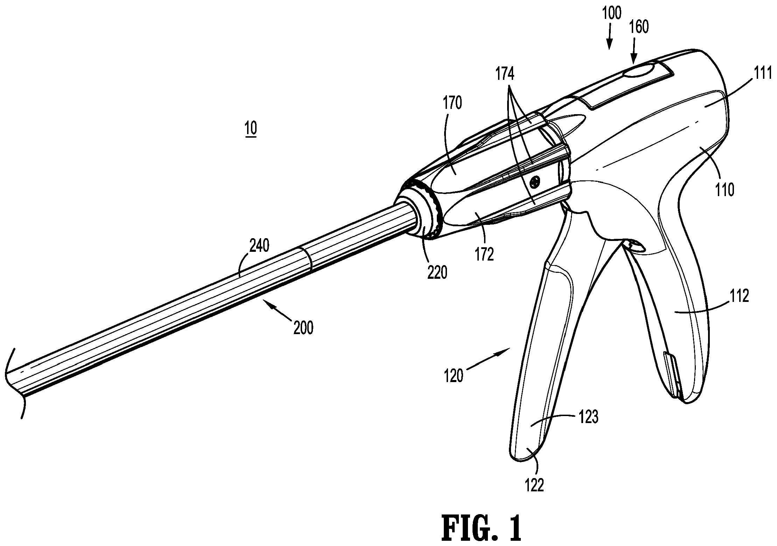

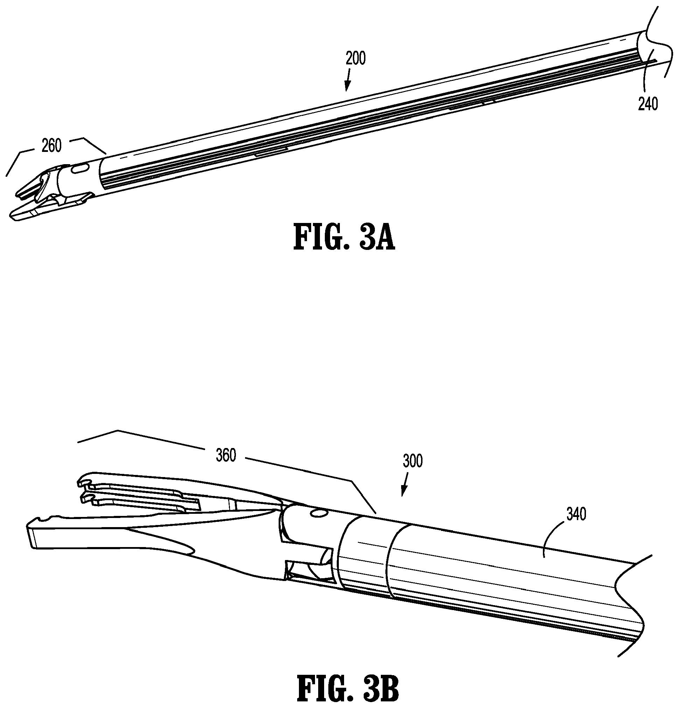

Turning to FIGS. 1-2, a surgical clip applier embodying the aspects and features of the present disclosure is shown generally identified by reference numeral 10. Surgical clip applier 10 generally includes a handle assembly 100 and a plurality of elongated assemblies 200, 300 (FIG. 3B) selectively connectable to handle assembly 100. Handle assembly 100 is configured to operate each of the plurality of elongated assemblies 200, 300 (FIG. 3B) upon connection thereto, and may be configured as a sterilizable, reusable component such that handle assembly 100 may be repeatedly used with different and/or additional elongated assemblies 200, 300 (FIG. 3B) during the course of one or more surgical procedures. The elongated assemblies 200, 300 (FIG. 3B) may be configured as single-use disposable components, limited-use disposable components, or reusable components, depending upon a particular purpose and/or the configuration of the particular elongated assembly. In either configuration, the need for multiple handle assemblies 100 is obviated and, instead, the surgeon need only select an appropriate elongated assembly 200, 300 (FIG. 3B) and connect that elongated assembly to handle assembly 100 in preparation for use.

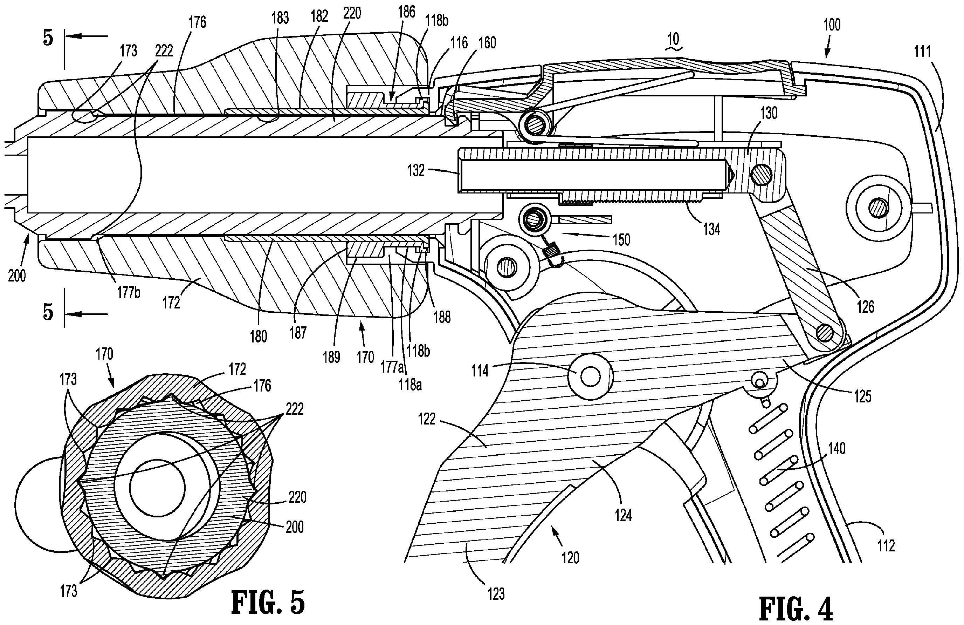

Handle assembly 100 generally includes a housing 110, an actuation mechanism 120 operably associated with housing 110, a ratchet mechanism 150 (FIG. 4) operably disposed within housing 110, a latch assembly 160 operably associated with housing 110, and a rotation knob assembly 170 operably coupled to a distal portion of housing 110. Housing 110 supports and/or encloses the operating components of handle assembly 100 and is detailed below. Actuation mechanism 120, detailed below, is configured to enable selective firing of one or more surgical clips (not shown) from the end effector of the attached elongated assembly. Rotation knob assembly 170 enables the selective rotation of the attached elongated assembly relative to housing 110, and is also detailed below.

Ratchet mechanical 150 enables ratcheting advancement of drive bar 130 (FIG. 4) of actuation mechanism 120, when an elongated assembly configured for ratcheting actuation is connected to handle assembly 100. Latch assembly 160 is configured to facilitate releasable locking engagement of the elongated assembly with handle assembly 100. Details of a suitable ratchet mechanism 150 and/or latch assembly 160 can be found in International Application No. PCT/CN2016/096666, filed on August 26, 2016, the entire contents of which is hereby incorporated herein by reference. Alternatively or additionally, ratchet mechanism 150 and/or latch assembly 160 may be configured as detailed in International Application No. PCT/CN2016/071178, filed on January 18, 2016, the entire contents of which is also hereby incorporated herein by reference.

With additional reference to FIGS. 3A and 3B, as noted above, handle assembly 100 is configured for use with different elongated assemblies such as, for example, elongated assembly 200 (FIGS. 1-3A) and elongated assembly 300 (FIG. 3B). Handle assembly 100, more specifically, is configured for both ratcheting use, e.g., in connection with elongated assembly 200 (FIGS. 1-3A), and non-ratcheting use, e.g., in connection with elongated assembly 300 (FIG. 3B). Elongated assemblies 200, 300 are described briefly below. A more detailed discussion of elongated assemblies, e.g., elongated assemblies 200, 300, configured for use with handle assembly 100 can be found in International Application Nos. PCT/CN2016/096666 and/or PCT/CN2016/071178, previously incorporated by reference herein in their entireties, and additionally or alternatively as in International Application No. PCT/CN2015/091603, filed on October 10, 2015, the entire contents of which is likewise hereby incorporated herein by reference.

Referring to FIGS. 1-3A, elongated assembly 200 is configured for ratcheting use and generally includes a proximal hub 220, an elongated shaft 240 extending distally from proximal hub 220, an end effector assembly 260 disposed towards a distal end portion of elongated shaft 240, and an inner drive assembly (not shown) operably coupled between handle assembly 100 and end effector assembly 260 when elongated assembly 200 is engaged with handle assembly 100 to enable the sequential firing of at least one surgical clip (not shown) about tissue. End effector assembly 260 of elongated assembly 200 may be configured to fire surgical clips similar to those shown and described in U.S. Patent Nos. 7,819,886 or 7,905,890, the entire contents of each of which is hereby incorporated herein by reference.

Proximal hub 220 of elongated assembly 200 defines a plurality of indexing protrusions 222 annularly disposed thereabout towards a distal end portion thereof. Indexing protrusions 222, as detailed below, are configured for slidable receipt within longitudinally-extending grooves 173 defined within outer knob 172 of rotation knob assembly 170 to rotationally fix proximal hub 220 of elongated assembly 200 relative to rotation knob assembly 170 upon insertion of proximal hub 220 therethrough (see also FIG. 5). As such, in use, rotation of outer knob 172 of rotation knob assembly 170 relative to housing 110 effects corresponding rotation of elongated assembly 200 relative to housing 110.

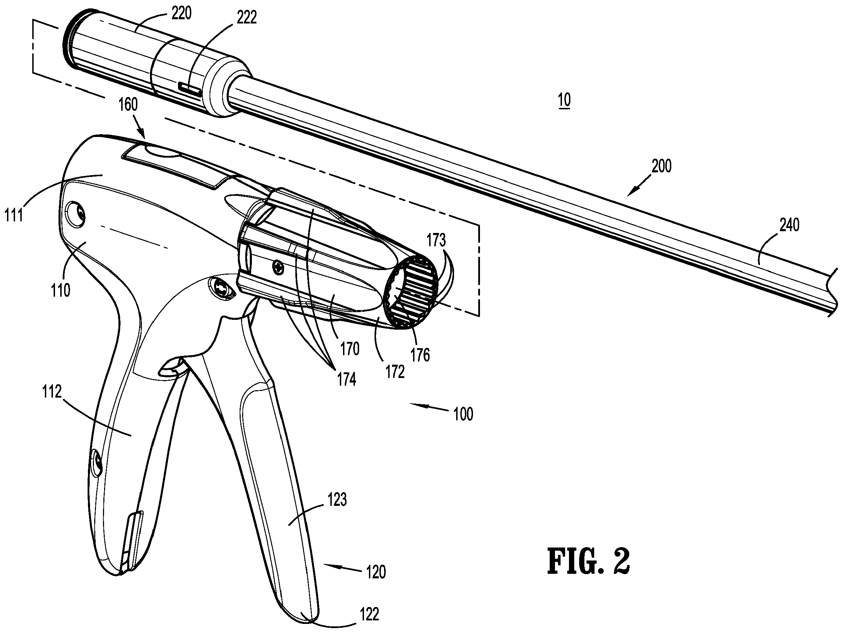

Referring to FIG. 3B, in conjunction with FIGS. 1 and 2, elongated assembly 300 is configured for non-ratcheting use and generally includes a proximal hub (not shown), an elongated shaft 340 extending distally from the proximal hub, an end effector assembly 360 disposed towards a distal end portion of elongated shaft 340, and an inner drive assembly (not shown) operably coupled between handle assembly 100 and end effector assembly 360 when elongated assembly 300 is engaged with handle assembly 100 to enable grasping and/or manipulation of tissue, retrieval of a surgical clip, and firing of the surgical clip about tissue. It is contemplated that end effector assembly 360 of elongated assembly 300 may be configured to fire surgical clips similar to those shown and described in U.S. Patent No. 4,834,096, the entire contents of which is hereby incorporated herein by reference.

The proximal hub (not shown) of elongated assembly 300 includes indexing protrusions similarly as detailed above with respect to proxy hub 220 of elongated assembly 200 (see FIG. 2) such that elongated assembly 300 is rotationally fix relative to rotation knob assembly 170 upon insertion of proximal hub 220 therethrough to enable rotation of elongated assembly 300 relative to housing 110 in response to rotation of outer knob 172 of rotation knob assembly 170 relative to housing 110.

Referring generally to FIGS. 1-3B, although exemplary elongated assemblies 200, 300 configured for ratcheting and non-ratcheting use, respectively, are detailed above, it is contemplated that various other elongated assemblies for performing various different surgical tasks and/or having various different configurations suitable for ratcheting or non-ratcheting use may likewise be utilized with handle assembly 100.

Turning to FIGS. 1, 2, and 4, housing 110 of handle assembly 100 may be formed from first and second housing halves that cooperate to define a body portion 111 and a fixed handle portion 112 depending from body portion 111. Body portion 111 of housing 110 includes an internal pivot post 114 extending transversely within body portion 111, and a distal nose 116 defining a distal opening 118a therethrough. A proximal end portion of a proximal hub of an elongated assembly, e.g., proximal hub 220 of elongated assembly 200 (FIGS. 1-3A) or the proximal hub (not shown) of elongated assembly 300 (FIG. 3B), is configured to extend at least partially through distal opening 118a of distal nose 116 of housing 110 when the elongated assembly 200 or 300 is engaged with handle assembly 100 (see FIG. 4). Distal nose 116 of body portion 111 of housing 110 further includes an annular recess 118b defined on an interior surface thereof surrounding distal opening 118a. Annular recess 118b is configured to receive proximal annular protrusion 188 of intermediate collar 186 of rotation knob assembly 170 to fixedly engage intermediate collar 186 with distal nose 116 of body portion 111 of housing 110, thereby rotatably engaging outer knob 172 and inner sleeve 180 of rotation knob assembly 170 with body portion 111 of housing 110. To this end, annular recess 118b and/or proximal annular protrusion 188 may include keying features or other suitable features or materials (not shown) to facilitate rotationally-locked engagement therebetween.

Actuation mechanism 120 is operably supported by housing 110 and includes a trigger 122, a linkage 126, a drive bar 130, and a biasing member 140. Trigger 122 includes a grasping portion 123, an intermediate pivot portion 124, and a proximal extension 125. Grasping portion 123 of trigger 122 extends downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture configured to receive pivot post 114 of housing 110 so as to enable pivoting of trigger 122 about pivot post 114 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

Proximal extension 125 of trigger 122 is disposed on an opposite side of intermediate pivot portion 124 and, thus, pivot post 114, as compared to grasping portion 123 of trigger 122. As such, pivoting of grasping portion 123 to rotate in one direction, e.g., proximally towards fixed handle portion 112, pivots proximal extension 125 to rotate in the opposite direction, e.g., distally. Proximal extension 125 of trigger 122 is pivotably coupled to the proximal end of linkage 126. Biasing member 140 is secured at either end and extends between proximal extension portion 125 of trigger 122 and a support (not shown) disposed within fixed handle portion 112 of housing 110. Pivoting of grasping portion 123 towards the actuated position elongates biasing member 140 storing energy therein such that, upon release of grasping portion 123, grasping portion 123 is returned towards the un-actuated position under the bias of biasing member 140. Although illustrated as an extension coil spring, biasing member 140 may define any suitable configuration for biasing grasping portion 123 of trigger 122 towards the un-actuated position.

As noted above, linkage 126 is coupled at its proximal end to proximal extension portion 125 of trigger 122. Linkage 126 is also pivotably coupled at its distal end to a proximal end of drive bar 130. As a result of this configuration, pivoting of grasping portion 123 of trigger 122 towards the actuated position urges proximal extension portion 125 of trigger 122 distally which, in turn, urges linkage 126 distally to, in turn, urge drive bar 130 distally.

Drive bar 130 is slidable through body portion 111 of housing 110, in response to actuation of trigger 122, to urge a distal end portion 132 of drive bar 130 into contact with a proximal actuator of an inner drive assembly (not shown) of an elongated assembly, e.g., elongated assembly 200 (FIGS. 1-3A) or elongated assembly 300 (FIG. 3B), engaged with handle assembly 100 to fire a surgical clip supported at the end effector assembly of the elongated assembly. Drive bar 130, more specifically, is slidable from an un-actuated, proximal position, corresponding to the un-actuated position of grasping portion 123 of trigger 122, to an actuated, distal position, corresponding to the actuated position of grasping portion 123 of trigger 122, in order to urge the proximal actuator of the inner drive assembly (not shown) of the elongated assembly distally to fire a surgical clip supported at the end effector assembly of the elongated assembly.

Drive bar 130 may further include a ratchet rack 134 extending along at least a portion of an underside surface thereof. Ratchet rack 134 is configured to selectively interface with ratchet mechanism 150 to enable advancement of drive bar 130 in either a ratcheting condition or a non-ratcheting condition. Ratchet rack 134 and ratchet mechanism 150, as noted above, may be configured similarly as described in, for example, International Application No. PCT/CN2016/096666 or International Application No. PCT/CN2016/071178, each of which was previously incorporated by reference herein.

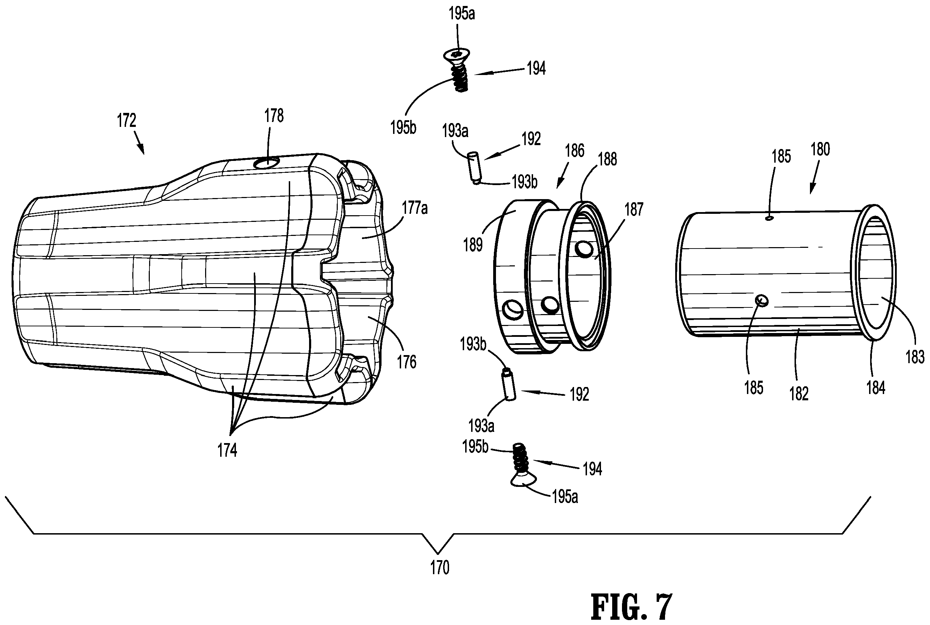

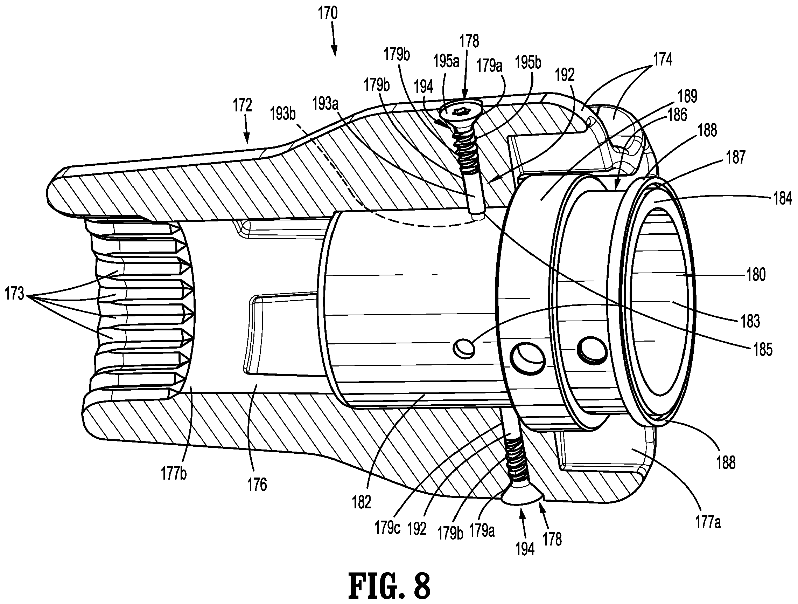

With reference to FIGS. 4-8, as noted above, rotation knob assembly 170 is coupled to distal nose 116 of body portion 111 of housing 110 and is configured to receive the proximal hub of the elongated assembly, e.g., proximal hub 220 of elongated assembly 200, coupled to handle assembly 100 in fixed rotational engagement therewith to enable selective rotation of elongated assembly 200 relative to housing 110 upon rotation of outer knob 172 of rotation knob assembly 170 relative to housing 110. Rotation knob assembly 170 includes outer knob 172, inner sleeve 180, and intermediate collar 186. Rotation knob assembly 170 further includes a pair of stepped pins 192 and a pair of screws 194. Outer knob 172 and inner sleeve 180 are fixedly engaged to one another via stepped pins 192 and screws 194, as detailed below, and rotatable relative to intermediate collar 186 which is disposed therebetween.

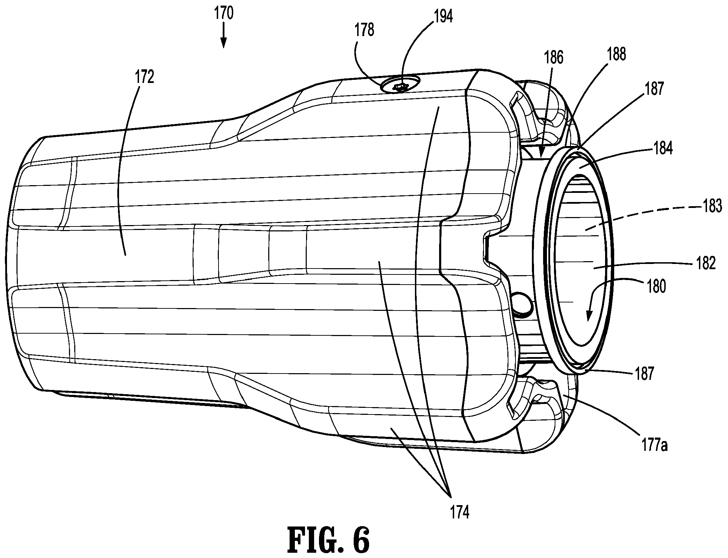

Referring to FIGS. 6-8, outer knob 172 of rotation knob assembly 170 may be formed from a polymeric material, e.g., a biocompatible, sterilizable plastic, or other suitable material, via molding or other suitable process and defines a cone shaped-configuration tapering in diameter from a proximal end portion to a distal end portion thereof, although other suitable configurations are also contemplated. Outer knob 172 includes a plurality of flutes 174 arranged radially about the exterior thereof to facilitate grasping or gripping outer knob 172 at any rotational orientation to enable rotation thereof.

Outer knob 172 of rotation knob assembly 170 further includes a longitudinally-extending lumen 176 defined therethrough between the proximal and distal ends thereof and a plurality of transverse apertures 178, e.g., a pair of opposed transverse apertures, defined through outer knob 172 from the exterior of outer knob 172 into communication with longitudinally-extending lumen 176 of outer knob 172. Longitudinally-extending lumen 176 includes an enlarged-diameter proximal portion 177a and a distal portion 177b. Distal portion 177b of longitudinally-extending lumen 176 includes grooves 173 disposed towards the distal end thereof which, as noted above, enable fixed rotational engagement of proximal hub 220 of elongated assembly 200 relative to outer knob 172 of rotation knob assembly 170 upon insertion of proximal hub 220 therethrough (see FIGS. 2, 4, and 5).

Each transverse aperture 178 of outer knob 172 includes a seat 179a disposed on the outwardly-facing end thereof, a threaded portion 179b extending inwardly from seat 179a, and a smooth or non-threaded portion 179c extending inwardly from threaded portion 179b to the inwardly-facing end of the transverse aperture 178.

Inner sleeve 180 of rotation knob assembly 170 may be formed from a metal, e.g., stainless steel, or other suitable material, and includes a body 182 defining a cylindrical configuration including a lumen 183 extending longitudinally therethrough, an outwardly-extending annular lip 184 disposed at the proximal end of body 182, and a plurality of transverse apertures 185 equally-spaced about and defined through body 182 of inner sleeve 180 from the exterior of body 182 into communication with lumen 183 of body 182. Thus, transverse apertures 185 define a depth equal to a thickness of body 182. Transverse apertures 185 of inner sleeve 180 each define a diameter smaller than a diameter of smooth portions 179c of transverse apertures 178 of outer knob 172.

Intermediate collar 186 of rotation knob assembly 180 may be formed from a metal, e.g., stainless steel, or other suitable material, and is configured for positioning about inner sleeve 180 within enlarged-diameter proximal portion 177a of longitudinally-extending lumen 176 of outer knob 172. Intermediate collar 186 defines a cylindrical configuration including a lumen 187 extending longitudinally therethrough, a proximal annular protrusion 188 extending outwardly therefrom at the proximal end thereof, and a distal annular protrusion 189 extending outwardly therefrom at the distal end thereof. As noted above, intermediate collar 186 is disposed between outer knob 172 and inner sleeve 180. Intermediate collar 186 is longitudinally retained relative to and between outer knob 172 and inner sleeve 180 between annular lip 184 of inner sleeve 180 and the shoulder defined at the interface between enlarged-diameter proximal portion 177a of longitudinally-extending lumen 176 of outer knob 172 and distal portion 177b of longitudinally-extending lumen 176 of outer knob 172. Intermediate collar 186 defines a length less than a length of inner sleeve 180 such that inner sleeve 180 extends distally from intermediate collar 186. Transverse apertures 185 of inner sleeve 180 are defined through the portion of inner sleeve that extends distally from intermediate collar 186.

Proximal annular protrusion 188 of intermediate collar 186, as noted above, is configured for receipt within annular recess 118b of distal nose 116 of body portion 111 of housing 110, thereby engaging intermediate collar 186 with body portion 111 of housing 110 (see FIG. 4) and, thus, coupling outer knob 172 of rotation knob assembly 170 about distal nose 116 of body portion 111 of housing 110. However, while intermediate collar 186 is fixed relative to housing 110, outer knob 172 and inner sleeve 180 are fixed to one another and together rotatable about and relative to intermediate collar 186 and, thus, relative to housing 110, e.g., to enable rotation of elongated assembly 200 relative to housing 110 (see FIGS. 2 and 4). Distal annular protrusion 189 of intermediate collar 186 defines a bearing surface about which outer knob 172 rotates, facilitating smooth rotation of outer knob 172 relative to housing 110.

Continuing with reference to FIGS. 6-8, stepped pins 192 and screws 194 fix outer knob 172 and inner sleeve 180 to one another to enable outer knob 172 and inner sleeve 180 to rotate together relative to intermediate collar 186 and housing 110 (see also FIG. 4). Each stepped pin 192, more specifically, includes a body portion 193a and tip portion 193b having a diameter less than the body portion 193a, thus defining a step therebetween. The diameter of the tip portion 193b of each stepped pin 192 generally approximates the diameter of the transverse apertures 185 of inner sleeve 180 to enable tip portions 193b to be received within transverse apertures 185 without significant play therebetween. Further, tip portions 193b define lengths equal to or less than the lengths of transverse apertures 185 (and, thus, the thickness of body 182 of inner sleeve 180) such that tip portions 193b may be received within transverse apertures 185 without extending into lumen 183 of inner sleeve 180, thus not interfering with an elongated assembly 200 (FIG. 4) inserted therethrough.

Body portions 193a of stepped pins 192 each define a diameter greater than the diameter of transverse apertures 185 of inner sleeve 180 and generally approximating the diameter of smooth portions 179c of transverse apertures 178 of outer knob 172 to enable body portions 193a to be received within smooth portions 179c of transverse apertures 178 of outer knob 172 without significant play therebetween and to inhibit body portions 193a from extending into transverse apertures 185 of inner sleeve 180. Stepped pins 192, led by tip portions 193b, may be inserted through the outwardly-facing ends of transverse apertures 178 of outer knob 172 through seats 179a and threaded portions 179b into position with body portions 193a of stepped pins 192 disposed within smooth portions 179c of transverse apertures 178 of outer knob 172 and tip portions 193b extending into transverse apertures 185 of inner sleeve 180. In this manner, stepped pins 192 fix inner sleeve 180 and outer knob 172 relative to one another.

Screws 194 are configured to retain stepped pins 192 in position to thereby retain inner sleeve 180 and outer knob 172 in fixed engagement with one another. Screws 194, more specifically, each include a head 195a and a threaded shank 195b extending from the head 195a. Threaded shanks 195b of screws 194 are configured for threaded engagement within threaded portions 179b of transverse apertures 178 of outer knob 172 until heads 195a are seated within seats 179a of transverse apertures 178 and the free ends of threaded shanks 195b abut stepped pins 192, thereby retaining stepped pins 192 in position fixing inner sleeve 180 and outer knob 172 with one another.

Referring generally to FIGS. 1, 2, and 4, in conjunction with FIGS. 6-8, insertion and engagement of an elongated assembly, e.g., elongated assembly 200, with handle assembly 100 and use of the same are described. In order to engage elongated assembly 200 with handle assembly 100, proximal hub 220 of elongated assembly 200 is inserted through the distal opening of outer knob 172 of rotation knob assembly 170 into distal portion 177a of longitudinally-extending lumen 176 of outer knob 172. Proximal hub 220 is advanced further proximally into lumen 183 of inner sleeve 180 and, eventually, into distal nose 116 of housing 110 wherein latch assembly 160 cams over the proximal end of proximal hub 220 and into engagement therewith to thereby rotatably engage proximal hub 220 relative to housing 110. Upon insertion of proximal hub 220 through rotation knob assembly 170, as noted above, indexing protrusions 222 of proximal hub 220 are received within longitudinally-extending grooves 173 of outer knob 172 to rotationally fix proximal hub 220 relative to outer knob 172 (see FIG. 5).

With elongated assembly 200 engaged with handle assembly 100 as detailed above, handle assembly 100 may be manipulated and/or outer knob 172 rotated to position end effector 260 (FIG. 3A) of elongated assembly 200 about tissue to be treated. Once end effector 260 is positioned as desired, trigger 122 is pivoted towards fixed handle portion 112 of housing 110 to urge linkage 126 distally which, in turn, urges drive bar 130 distally through housing 110 to drive the proximal actuator of the inner drive assembly (not shown) of elongated assembly 200 distally through elongated assembly 200 to fire and form a surgical clip from end effector assembly 260 (FIG. 3A) about tissue. The above may be repeated to fire and form several surgical clips about tissue, as necessary.

In order to disengage elongated assembly 200 from handle assembly 100, e.g., for cleaning and/or sterilization, or to replace elongated assembly 200 with another endoscopic assembly, latch assembly 160 is depressed inwardly into housing 110 to disengage proximal hub 220 of elongated assembly 200, thus enabling proximal hub 220 to be withdrawn distally from housing 110 and rotation knob assembly 170.

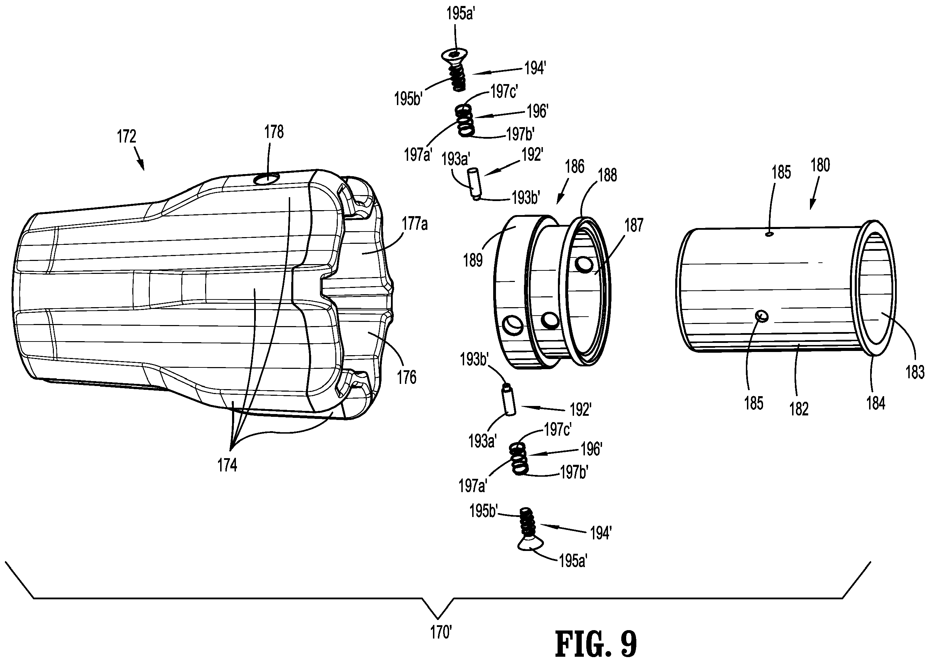

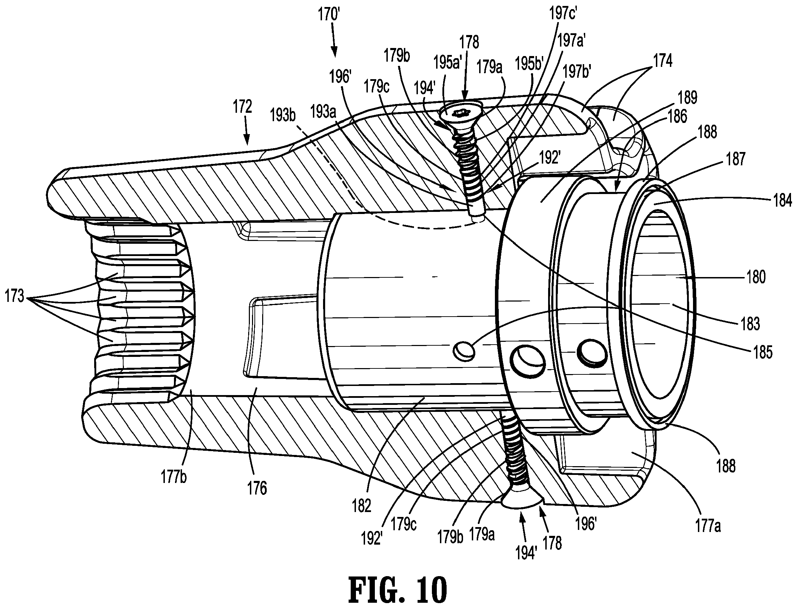

With reference to FIGS. 9 and 10, a rotation knob assembly 170' is couplable to distal nose 116 of body portion 111 of housing 110 and is configured to receive the proximal hub of the elongated assembly, e.g., proximal hub 220 of elongated assembly 200, in fixed rotational engagement therewith to enable selective rotation of elongated assembly 200 relative to housing 110 upon rotation of outer knob 172 of rotation knob assembly 170' relative to housing 110 (see FIG. 4). Rotation knob assembly 170' is substantially similar to rotation knob assembly 170 of FIGS. 6-8 and will only be described herein with respect to the differences therebetween. Rotation knob assembly 170' includes outer knob 172, inner sleeve 180, and intermediate collar 186. Rotation knob assembly 170' further includes a pair of stepped pins 192', a pair of screws 194', and a pair of springs 196'. Outer knob 172 and inner sleeve 180 are fixedly engaged to one another via stepped pins 192', screws 194', and springs 196', as detailed below, and rotatable relative to intermediate collar 186 which is disposed therebetween.

The stacked configuration of stepped pins 192', springs 196', and screws 194' fix outer knob 172 and inner sleeve 180 to one another to enable outer knob 172 and inner sleeve 180 to rotate together relative to intermediate collar 186 and housing 110 (see also FIG. 4). Each stepped pin 192' includes a body portion 193a' and tip portion 193b' having a diameter less than the body portion 193a', thus defining a step therebetween. The diameter of the tip portion 193b' of each stepped pin 192' generally approximates the diameter of the transverse apertures 185 of inner sleeve 180 to enable tip portions 193b' to be received within transverse apertures 185 without significant play therebetween. Further, tip portions 193b' define lengths equal to or less than the lengths of transverse apertures 185 (and, thus, the thickness of body 182 of inner sleeve 180) such that tip portions 193b' may be received within transverse apertures 185 without extending into lumen 183 of inner sleeve 180, thus not interfering with an elongated assembly 200 (FIG. 4) inserted therethrough.

Body portions 193a' of stepped pins 192' each define a diameter greater than the diameter of transverse apertures 185 of inner sleeve 180 and generally approximating the diameter of smooth portions 179c of transverse apertures 178 of outer knob 172 to enable body portions 193a' to be received within smooth portions 179c of transverse apertures 178 of outer knob 172 without significant play therebetween and to inhibit body portions 193a' from extending into transverse apertures 185 of inner sleeve 180. Stepped pins 192', led by tip portions 193b', may be inserted through the outwardly-facing ends of transverse apertures 178 of outer knob 172, through seats 179a and threaded portions 179b, and into position with body portions 193a' of stepped pins 192' disposed within smooth portions 179c of transverse apertures 178 of outer knob 172 and tip portions 193b' extending into transverse apertures 185 of inner sleeve 180. In this manner, stepped pins 192' fix inner sleeve 180 and outer knob 172 relative to one another.

Springs 196' are configured for positioning between stepped pins 192' and screws 194', and are deflectable or compressible to compensate for length variations of stepped pins 192' and/or screws 194'. Each spring 196' includes a coiled or helical body 197a' having a first end 197b' and a second end 197c'. The diameter of the helical body 197a' of each spring 196' generally approximates the diameter of smooth portions 179c of transverse apertures 178 of outer knob 172 to enable helical body 197a' to be received within smooth portions 179c of transverse apertures 178 without significant play therebetween. Springs 196' may be inserted into transverse apertures 178 of outer knob 172, after insertion of stepped pins 192' as described above, and into position with first ends 197b' of springs 196' adjacent to (e.g., abutting or touching) free ends of body portions 193a' of stepped pins 192' and extending axially therefrom within smooth portions 179c of transverse apertures 178.

Screws 194' each include a head 195a' and a threaded shank 195b' extending from the head 195a'. Threaded shanks 195b' of screws 194' are configured for threaded engagement within threaded portions 179b of transverse apertures 178 of outer knob 172 until heads 195a' are seated within seats 179a of transverse apertures 178 and the free ends of threaded shanks 195b' are adjacent to (e.g., abut or touch) or engage the second ends 197c' of springs 196' to retain stepped pins 192' in position fixing inner sleeve 180 and outer knob 172 with one another. Screws 194' may compress springs 196' between threaded shanks 195b' of screws 194' and body portions 193a' of stepped pins 192' to maintain stepped pins 192', springs 196', and screws 194' in a compressed state within transverse apertures 178 of outer knob 172 and ensure inner sleeve 180 and outer knob 172 are in fixed or locked engagement with one another and/or to reduce error which may be introduced by length tolerance stack-up of stepped pins 192' and screws 194'.

While the outer knob is described as having transverse apertures having threaded and smooth portions, other configurations are additionally or alternatively possible. For example, the transverse apertures may have smooth portions extending a majority or the entire length thereof. The threaded shanks of the screws may be configured as thread-forming or thread-cutting screws to form mating threads in the smooth portions of the transverse apertures of the outer knob to retain the screws therein and minimize loosening of the screws.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A rotation knob assembly for a surgical instrument, the rotation knob assembly comprising:
   an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
   an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
   at least one stepped pin including a body portion disposed within the smooth portion of the at least one outer knob transverse aperture and a tip portion disposed within the at least one inner sleeve transverse aperture to fix the outer knob and the inner sleeve with one another; and
   at least one screw threadingly engaged within the threaded portion of the at least one outer knob transverse aperture to retain the at least one stepped pin in position, thereby retaining the outer knob and the inner sleeve fixed with one another.
2. The rotation knob assembly according to paragraph 1, further comprising an intermediate collar disposed between the outer knob and the inner sleeve.
3. The rotation knob assembly according to paragraph 2, wherein the intermediate collar is fixed and wherein the outer knob and the inner sleeve are together rotatable relative to the intermediate collar.
4. The rotation knob assembly according to paragraph 2, wherein the outer knob lumen includes a proximal lumen portion and a distal lumen portion, the proximal lumen portion defining a diameter greater than a diameter of the distal lumen portion, the proximal lumen portion housing the intermediate collar therein.
5. The rotation knob assembly according to paragraph 1, further comprising at least one spring disposed within the at least one outer knob transverse aperture, the at least one spring compressed between the at least one stepped pin and the at least one screw.
6. The rotation knob assembly according to paragraph 1, wherein the at least one outer knob transverse aperture further defines a seat and wherein the at least one screw includes a head configured to be received within the seat.
7. A handle assembly of a surgical instrument, comprising:
   a housing defining a body portion, a fixed handle portion depending from the body portion, and a distal nose extending distally from the body portion;
   a drive assembly disposed within the housing;
   a trigger pivotably connected to the housing and operably associated with the drive assembly, the trigger movable relative to the fixed handle portion of the housing from an un-actuated position to an actuated position to actuate the drive assembly; and
   a rotation knob assembly extending distally from the distal nose of the housing, the rotation knob assembly including:
      an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
      an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
      at least one stepped pin including a body portion disposed within the smooth portion of the at least one outer knob transverse aperture and a tip portion disposed within the at least one inner sleeve transverse aperture to fix the outer knob and the inner sleeve with one another; and
      at least one screw threadingly engaged within the threaded portion of the at least one outer knob transverse aperture to retain the at least one stepped pin in position, thereby retaining the outer knob and the inner sleeve fixed with one another.
8. The handle assembly according to paragraph 7, wherein the rotation knob assembly further comprises an intermediate collar disposed between the outer knob and the inner sleeve.
9. The handle assembly according to paragraph 8, wherein the intermediate collar is fixed relative to the distal nose of the housing and wherein the outer knob and the inner sleeve are together rotatable relative to the intermediate collar and the distal nose of the housing.
10. The handle assembly according to paragraph 8, wherein the outer knob lumen includes a proximal lumen portion and a distal lumen portion, the proximal lumen portion defining a diameter greater than a diameter of the distal lumen portion, the proximal lumen portion housing the intermediate collar therein.
11. The handle assembly according to paragraph 7, wherein the rotation knob assembly further comprises at least one spring disposed within the at least one outer knob transverse aperture, the at least one spring compressed between the at least one stepped pin and the at least one screw.
12. The handle assembly according to paragraph 7, wherein the at least one outer knob transverse aperture further defines a seat and wherein the at least one screw includes a head configured to be received within the seat.
13. A surgical instrument, comprising:
   a handle assembly; and
   an elongated assembly extending distally from the handle assembly and supporting an end effector assembly at a distal end portion thereof;
   wherein the handle assembly includes:
      a housing defining a body portion, a fixed handle portion depending from the body portion, and a distal nose extending distally from the body portion;
      a drive assembly disposed within the housing;
      a trigger pivotably connected to the housing and operably associated with the drive assembly, the trigger movable relative to the fixed handle portion of the housing from an un-actuated position to an actuated position to actuate the drive assembly; and
      a rotation knob assembly extending distally from the distal nose of the housing and disposed about the elongated assembly, the rotation knob assembly including:
         an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
         an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
         at least one stepped pin including a body portion disposed within the smooth portion of the at least one outer knob transverse aperture and a tip portion disposed within the at least one inner sleeve transverse aperture to fix the outer knob and the inner sleeve with one another; and
         at least one screw threadingly engaged within the threaded portion of the at least one outer knob transverse aperture to retain the at least one stepped pin in position, thereby retaining the outer knob and the inner sleeve fixed with one another.
14. The surgical instrument according to paragraph 13, wherein the rotation knob assembly further comprises an intermediate collar disposed between the outer knob and the inner sleeve.
15. The surgical instrument according to paragraph 14, wherein the intermediate collar is fixed relative to the distal nose of the housing and wherein the outer knob and the inner sleeve are together rotatable relative to the intermediate collar and the distal nose of the housing.
16. The surgical instrument according to paragraph 14, wherein the outer knob lumen includes a proximal lumen portion and a distal lumen portion, the proximal lumen portion defining a diameter greater than a diameter of the distal lumen portion, the proximal lumen portion housing the intermediate collar therein.
17. The surgical instrument according to paragraph 13, wherein the outer knob defines a plurality of grooves disposed on an interior surface thereof surrounding the outer knob lumen, at least one groove of the plurality of grooves configured to receive a corresponding indexing protrusion of the elongated assembly to rotationally fix the elongated assembly relative to the outer knob.
18. The surgical instrument according to paragraph 13, wherein the rotation knob assembly further comprises at least one spring disposed within the at least one outer knob transverse aperture, the at least one spring compressed between the at least one stepped pin and the at least one screw.
19. The surgical instrument according to paragraph 13, wherein the at least one outer knob transverse aperture further defines a seat and wherein the at least one screw includes a head configured to be received within the seat.
20. The surgical instrument according to paragraph 13, further comprising a latch assembly operably associated with the housing, wherein the latch assembly is configured to releasably engage the elongated assembly with the housing.

## Claims

1. A rotation knob assembly for a surgical instrument, the rotation knob assembly comprising:
an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
at least one stepped pin including a body portion disposed within the smooth portion of the at least one outer knob transverse aperture and a tip portion disposed within the at least one inner sleeve transverse aperture to fix the outer knob and the inner sleeve with one another; and
at least one screw threadingly engaged within the threaded portion of the at least one outer knob transverse aperture to retain the at least one stepped pin in position, thereby retaining the outer knob and the inner sleeve fixed with one another.

2. The rotation knob assembly according to claim 1, further comprising an intermediate collar disposed between the outer knob and the inner sleeve.

3. The rotation knob assembly according to claim 2, wherein the intermediate collar is fixed and wherein the outer knob and the inner sleeve are together rotatable relative to the intermediate collar; and/or wherein the outer knob lumen includes a proximal lumen portion and a distal lumen portion, the proximal lumen portion defining a diameter greater than a diameter of the distal lumen portion, the proximal lumen portion housing the intermediate collar therein.

4. The rotation knob assembly according to any preceding claim, further comprising at least one spring disposed within the at least one outer knob transverse aperture, the at least one spring compressed between the at least one stepped pin and the at least one screw.

5. The rotation knob assembly according to any preceding claim, wherein the at least one outer knob transverse aperture further defines a seat and wherein the at least one screw includes a head configured to be received within the seat.

6. A handle assembly of a surgical instrument, comprising:
a housing defining a body portion, a fixed handle portion depending from the body portion, and a distal nose extending distally from the body portion;
a drive assembly disposed within the housing;
a trigger pivotably connected to the housing and operably associated with the drive assembly, the trigger movable relative to the fixed handle portion of the housing from an un-actuated position to an actuated position to actuate the drive assembly; and
a rotation knob assembly extending distally from the distal nose of the housing, the rotation knob assembly including:
an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
at least one stepped pin including a body portion disposed within the smooth portion of the at least one outer knob transverse aperture and a tip portion disposed within the at least one inner sleeve transverse aperture to fix the outer knob and the inner sleeve with one another; and
at least one screw threadingly engaged within the threaded portion of the at least one outer knob transverse aperture to retain the at least one stepped pin in position, thereby retaining the outer knob and the inner sleeve fixed with one another.

7. The handle assembly according to claim 6, wherein the rotation knob assembly further comprises an intermediate collar disposed between the outer knob and the inner sleeve.

8. The handle assembly according to claim 7, wherein the intermediate collar is fixed relative to the distal nose of the housing and wherein the outer knob and the inner sleeve are together rotatable relative to the intermediate collar and the distal nose of the housing; and/or wherein the outer knob lumen includes a proximal lumen portion and a distal lumen portion, the proximal lumen portion defining a diameter greater than a diameter of the distal lumen portion, the proximal lumen portion housing the intermediate collar therein.

9. The handle assembly according to any of claims 6 to 8, wherein the rotation knob assembly further comprises at least one spring disposed within the at least one outer knob transverse aperture, the at least one spring compressed between the at least one stepped pin and the at least one screw.

10. The handle assembly according to any of claims 6 to 9, wherein the at least one outer knob transverse aperture further defines a seat and wherein the at least one screw includes a head configured to be received within the seat.

11. A surgical instrument, comprising:
a handle assembly; and
an elongated assembly extending distally from the handle assembly and supporting an end effector assembly at a distal end portion thereof;
wherein the handle assembly includes:
a housing defining a body portion, a fixed handle portion depending from the body portion, and a distal nose extending distally from the body portion;
a drive assembly disposed within the housing;
a trigger pivotably connected to the housing and operably associated with the drive assembly, the trigger movable relative to the fixed handle portion of the housing from an un-actuated position to an actuated position to actuate the drive assembly; and
a rotation knob assembly extending distally from the distal nose of the housing and disposed about the elongated assembly, the rotation knob assembly including:
an outer knob defining an outer knob lumen extending longitudinally therethrough and at least one outer knob transverse aperture extending transversely therethrough into communication with the outer knob lumen, the at least one outer knob transverse aperture including a threaded portion and a smooth portion, the smooth portion defining a first diameter;
an inner sleeve disposed within the outer knob lumen, the inner sleeve including a body defining an inner sleeve lumen extending longitudinally therethrough and at least one inner sleeve transverse aperture extending transversely through the body into communication with the inner sleeve lumen, the at least one inner sleeve transverse aperture defining a second diameter less than the first diameter;
at least one stepped pin including a body portion disposed within the smooth portion of the at least one outer knob transverse aperture and a tip portion disposed within the at least one inner sleeve transverse aperture to fix the outer knob and the inner sleeve with one another; and
at least one screw threadingly engaged within the threaded portion of the at least one outer knob transverse aperture to retain the at least one stepped pin in position, thereby retaining the outer knob and the inner sleeve fixed with one another.

12. The surgical instrument according to claim 11, wherein the rotation knob assembly further comprises an intermediate collar disposed between the outer knob and the inner sleeve; and/or wherein the intermediate collar is fixed relative to the distal nose of the housing and wherein the outer knob and the inner sleeve are together rotatable relative to the intermediate collar and the distal nose of the housing; and/or wherein the outer knob lumen includes a proximal lumen portion and a distal lumen portion, the proximal lumen portion defining a diameter greater than a diameter of the distal lumen portion, the proximal lumen portion housing the intermediate collar therein.

13. The surgical instrument according to any of claims 11 to 12 wherein the outer knob defines a plurality of grooves disposed on an interior surface thereof surrounding the outer knob lumen, at least one groove of the plurality of grooves configured to receive a corresponding indexing protrusion of the elongated assembly to rotationally fix the elongated assembly relative to the outer knob.

14. The surgical instrument according to any of claims 11 to 13 wherein the rotation knob assembly further comprises at least one spring disposed within the at least one outer knob transverse aperture, the at least one spring compressed between the at least one stepped pin and the at least one screw.

15. The surgical instrument according to any of claims 11 to 14, wherein the at least one outer knob transverse aperture further defines a seat and wherein the at least one screw includes a head configured to be received within the seat; and/or further comprising a latch assembly operably associated with the housing, wherein the latch assembly is configured to releasably engage the elongated assembly with the housing.
